# EUROPÄISCHE PATENTANMELDUNG

(11) **EP 3 352 106 A1**
(43) Veröffentlichungstag der Anmeldung: **25.07.2018**
(21) Anmeldenummer: 17152427.5
(22) Anmeldetag: 20.01.2017
(51) Int. Cl.: G06F 19/00, G06Q 50/22

(54) **VERFAHREN ZUM ÜBERWACHEN DES ZUSTANDS VON INSTRUMENTEN UND WERKZEUGEN FÜR CHIRURGISCHE EINGRIFFE UND ÄHNLICHE EINSÄTZE**

(71) Anmelder: Leuthold, Stefan, 8049 Zurich (CH)
(72) Erfinder: Leuthold, Stefan, 8049 Zurich (CH)
(74) Vertreter: Luchs, Willi

(57) **Zusammenfassung**

Die Erfindung betrifft ein Verfahren zum Überwachen des Zustands von Instrumenten/Werkzeugen für chirurgische Eingriffe und ähnliche Einsätze, wobei der Zustand der Instrumente/Werkzeuge anhand von für ihre Einsatzfähigkeit massgeblichen Parametern wie Temperatur, Feuchte, Impuls, EO-Konzentration, Strahlung, etc. vorzugsweise kontinuierlich mit in ihnen eingebauten Sensoren erfasst wird. Die von diesen erhaltenen Daten werden zeitgleich oder zeitversetzt in einer Datenverarbeitungseinrichtung anhand von instrumentenspezifischen Sollvorgaben vorzugsweise algorithmisch ausgewertet.

## Beschreibung

Die Erfindung betrifft ein Verfahren zum Überwachen des Zustands von Instrumenten und Werkzeugen für chirurgische Eingriffe und ähnliche Einsätze. Die Erfindung betrifft auch ein Instrument/Werkzeug zum Ausführen des vorgeschlagenen Verfahrens.

Besonders in der medizinischen Chirurgie ist der Zustand der verwendeten Instrumente und Werkzeuge einer der ausschlaggebenden Faktoren für das Gelingen der durchzuführenden Eingriffe. In diesem Zusammenhang ist es für Kliniken, Spitäler und andere Dienstleistungsbetriebe eine grosse Herausforderung, jederzeit den Zustand der eingesetzten Instrumente/Werkzeuge nachweisen zu können, um bei Operationen Fehlmanipulationen zu vermeiden, etwaige Fehlerquellen zu untersuchen, Keimvernichtungen nachzuweisen oder künftige Regulationsvorschriften (MDR, FDA etc.) einzuhalten.

Die Kontrolle des ordnungsgemässen Zustandes von chirurgischen Instrumenten und Werkzeugen erfolgt in der Praxis durch Inspektion derselben seitens des sie bereitstellenden Personals und der den Eingriff durchführenden Chirurgen, einschliesslich des assistierenden Personals. Es versteht sich von selbst, dass diese Verfahrensweise im Ergebnis vom subjektiven Urteil und der individuellen Sorgfalt des involvierten Fachpersonals abhängig ist. Diese Kontrollweise hat ausserdem den Nachteil, dass oft eine genaue Rekonstruktion des Zustandes der eingesetzten Instrumente/Werkzeuge im Nachhinein nicht mehr nachvollziehbar ist.

Der Erfindung liegt die Aufgabe zugrunde, diese Nachteile zu vermeiden und ein Verfahren der eingangs genannten Art zu schaffen, das jederzeit den tatsächlichen Zustand der benutzten Instrumente und Werkzeuge anzeigt und registriert, unabhängig von subjektiven Faktoren, wie etwa Wahrnehmungsfehler oder mangelnde Sorgfalt des sie bereitstellenden oder benutzenden Fachpersonals.

Diese Aufgabe ist erfindungsgemäss dadurch gelöst, dass der Zustand der Instrumente/Werkzeuge anhand von für ihre Einsatzfähigkeit massgeblichen Parametern vorzugsweise kontinuierlich mit in ihnen eingebauten Sensoren erfasst wird und die von diesen erhaltenen Daten zeitgleich oder zeitversetzt anhand von instrumentenspezifischen Sollvorgaben ausgewertet werden.

Auf diese Weise ist es möglich, bei chirurgischen Eingriffen jegliche Fehlmanipulationen der Instrumente/Werkzeuge wegen einer Fehlbeurteilung ihres Zustandes zu vermeiden, was sonst unter Umständen zur Folge haben kann, dass der Eingriff wiederholt werden muss, um die missratene Operation zu korrigieren, oder der Patient nachhaltig geschädigt werden kann.

Das erfindungsgemässe Verfahren ermöglicht den fachgerechten Gebrauch der Instrumente/Werkzeuge, was bei Operationen eine Reduzierung der Fehlerquoten mit sich bringt. Und bei missratenen Eingriffen ermöglicht es auch die Fehlersuche anhand von verlässlichen Aufzeichnungen des Zustands der verwendeten Instrumente/Werkzeuge.

Die dem Verfahren zugrundeliegenden Parameter und ihre Sollvorgaben werden in Abhängigkeit von der Art und Funktion der Instrumente/Werkzeuge festgelegt, wobei in der Praxis Faktoren wie Temperatur, Feuchte, Impuls, Dehnung, Überspannung, Überstrom, EO-Konzentration, Strahlung und ähnliches in Frage kommen.

Die Erfindung sieht weiterhin vor, dass die Auswertung der von den Instrumenten/Werkzeugen erhaltenen Daten je nach Art des Eingriffs ein- oder mehrmals, während, vor und/oder nach dem Einsatz in einem mit den Sensoren durch Datenübertragungsmittel verbundenen Empfänger durchgeführt wird.

Zwecks einer schnellen Beurteilung der gewonnenen Aufzeichnungen sieht die Erfindung auch vor, dass die Auswertungsergebnisse durch ein geeignetes Mittel als Schnittstelle zwischen dem Fachpersonal und der Hardware, zum Beispiel einem Bildschirmgerät oder ähnlichem, visualisiert werden können.

Je nach Komplexität der chirurgischen Aufgabe werden die von den Instrumenten/Werkzeugen erhaltenen Daten entweder am Instrument/Werkzeug selber oder auf einer Datenbank des Empfängers vorzugsweise aggregiert ausgewertet.

Im letzteren Fall werden die Daten erfindungsgemäss von einem Computer oder einer ähnlichen zentralen Datenverarbeitungseinrichtung aufgezeichnet, gespeichert und vorzugsweise algorithmisch ausgewertet, indem sie dort mit den jeweils eingegebenen Sollvorgaben verglichen werden. Die zentrale Datenverarbeitung ermöglicht nicht nur eine individuelle Beurteilung des Zustandes einzelner Geräte, sondern auch eine interdisziplinäre Übersicht über den Gesamtzustand der verwendeten Ausrüstung.

Für die Auswertung der von den Instrumenten/Werkzeugen gewonnenen Daten ist es zweckmässig, dass der Computer zusätzlich auch weitere einsatzspezifische Daten wie Einsatzort, Einsatztermin oder Einsatzdauer des Eingriffes berücksichtigt.

Der Computer kann auch erfindungsgemäss eine Überlastung der Instrumente/Werkzeuge durch Aufzeichnung der auf sie während des Einsatzes wirkenden Spannungen und Kräfte registrieren.

Es ist auch zum Vermeiden von Operationsfehlern zweckmässig, wenn der Computer ebenfalls eine Beschädigung oder gar das Ende der Lebensdauer der Instrumente/Werkzeuge registriert und dies durch ein optisches und/oder akustisches Signal angezeigt wird.

Es ist auch in diesem Sinne zweckmässig, wenn der Computer mittels eines 3D-Beschleunigungsmessers das Herunterfallen der Instrumente/Werkzeuge aus der Hand des Operateurs oder zu Boden registriert.

Der Computer kann ebenfalls einen Sterilisations- oder Reinigungsvorgang durch Temperatur-, Feuchte- und EO-Konzentrationsmessung registrieren.

Das erfindungsgemässe Instrument/Werkzeug zum Ausüben des vorgeschlagenen Verfahrens zeichnet sich dadurch aus, dass in ihm miniaturisierte Sensoren eingebaut sind, die vorzugsweise kontinuierlich jeweils ein oder mehrere der zu berücksichtigenden Parameter erfassen. Diese Sensoren verändern nur minimal die konventionelle Ausgestaltung des Geräts. Sie können die von ihnen gewonnenen Daten auf den Empfänger via Funk mittels WLAN, Bluetooth, NFC und ähnlicher elektronischer Mittel übertragen.

Die Sensoren können entweder in einer Einkapselung oder unter einer Abdeckung des Instruments/Werkzeugs eingebaut sein. Diese Konstruktion ermöglicht auch den nachträglichen Einbau der Sensoren in konventionell ausgestalteten Geräten.

Die Erfindung ist nachfolgend anhand mehrerer Ausführungsbeispiele unter Bezugnahme auf die Zeichnung näher erläutert. Es zeigen:
- Fig. 1: Funktions- und Ablaufschema eines nach dem erfindungsgemässen Verfahren arbeitenden Systems,
- Fig. 2: ein erfindungsgemäss konstruiertes Instrument, in der Draufsicht dargestellt,
- Fig. 3: eine Einzelheit des Instruments aus Fig. 2 in der Ausführung mit einem eingekapselten Sensor, und
- Fig. 4: die Einzelheit gemäss Fig. 3 in der Ausführung mit einem unter einer Abdeckung angeordneten Sensor.

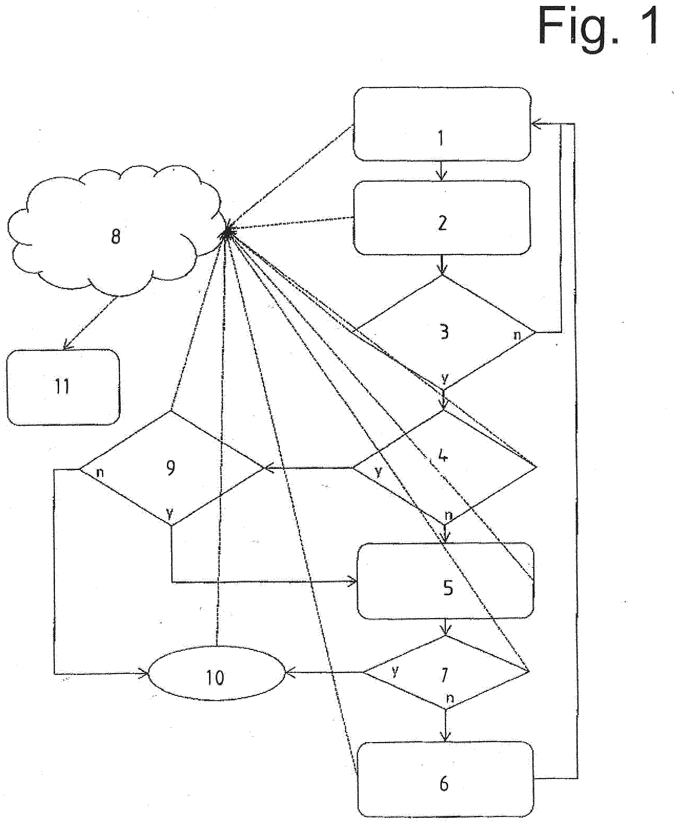

Das in Fig. 1 schematisch dargestellte Überwachungssystem dient zum laufenden Überwachen des Zustandes von chirurgischen bzw. medizinalen Instrumenten, wie Spritzen, Zangen, Scheren Messer, etc., die je nach Art des Eingriffs unterschiedlichen Einsatzbedingungen ausgesetzt sind und somit während der Operation unterschiedliche Beanspruchungen erfahren.

Dieses Überwachungssystem umfasst mehrere Stationen 1 bis 7 mit unterschiedlichen Funktionen, die untereinander über eine Einrichtung in Gestalt einer Daten-Clouds 8 mit den zu überwachenden Instrumenten vernetzt sind. Diese Verbindungen sind in Fig. 1 mit durchgezogenen und punktierten Linien schematisch dargestellt.

Die Station 1 betrifft das Lagerregal des Magazins, in dem die zu überwachenden Instrumente fachgerecht aufbewahrt sind. Die Station 2 betrifft einen Operationsraum, in dem die Instrumente eingesetzt werden, indes die Stationen 3 bis 7 ihrerseits die beim Eingriff eingesetzten Instrumente betreffen, wobei sich die Stationen 4 bis 7 auf einzelne Merkmale beziehen, die für den Zustand der Instrumente relevant sind. So wird in Station 4 das Herunterfallen der Instrumente registriert, in Station 5 ihr Reinigungszustand, in Station 6 ihr Sterilisationszustand und in Station 7 das Ende der Lebensdauer des Instrumentes.

Weitere Stationen des Systems sind die Station 9 mit einer Einrichtung zum Visualisieren der via Daten-Clouds gewonnenen Messwerte sowie eine Station 10, in der verbrauchte Instrumente einem Recyclingprozess zugeführt werden.

Die Auswertung und Registrierung der in der Daten-Clouds 8 enthaltenen Daten erfolgt in einem oder mehreren Computern. Diese sind in Fig. 1 symbolisch mit der Station 11 angedeutet.

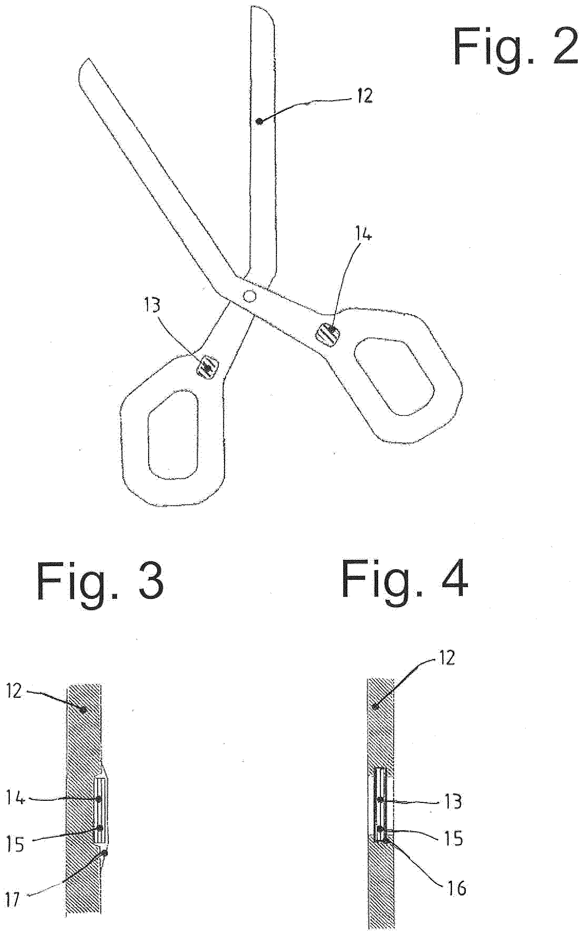

Das in Fig. 2 bis Fig. 4 gezeigte Instrument 12 zum Ausüben des erfindungsgemässen Überwachungsverfahrens ist mit zwei miniaturisierten Sensoren 13, 14 ausgerüstet, die gleichzeitig einen Datenspeicher, eine Stromversorgung und eine Datenübertragungseinheit 15 beinhalten. Solche Sensoren ermöglichen sowohl die individuelle Auswertung und Registrierung der Daten direkt am Gerät als auch die kollektive Auswertung und Registrierung in Verbindung mit den Daten anderer Instrumente oder Sensoren.

Im Ausführungsbeispiel nach Fig. 3 ist der Sensor 13 im Instrument 12 in einer Einkapselung 16 integriert, während in derjenigen nach Fig. 4 der Sensor 14 unter einer Abdeckung 17 angeordnet ist. Er kann somit leichter abgebaut und ausgetauscht werden. In beiden Fällen handelt es sich um RFID-Sensoren, mit welchen vorzugsweise verschiedene Daten feststellbar sind.

Beim beschriebenen Ausführungsbeispiel ist das zu überwachende Instrument 12 mit zwei Sensoren ausgestattet. Es könnte auch nur ein oder mehr als zwei Sensoren vorgesehen sein. Dies kann selbstverständlich je nach Art und Aufgabe des Instrumentes variieren.

Das erfindungsgemässe Überwachungssystem ist sowohl bei sogenannten Einmalgebrauch- bzw. Single use-Instrumenten als auch bei sogenannten Multi use-Instrumenten anwendbar. Bei ersteren kann beispielsweise die Beweisführung des Merkmals Sterilisation nachvollziehbar auf der Ebene jeder einzelnen Einheit belegt werden. Bei multi use-Instrumenten kann der Kreislauf ausgehend vom Inverkehrbringen, Gebrauch, Reinigung, Sterilisation und erneuter Verwendung überwacht werden.

Mit dem beschriebenen Überwachungssystem kann der Operateur den tatsächlichen Zustand der Instrumente während der Dauer des Einsatzes überwachen und mögliche Überbeanspruchungen erkennen, die eine Gefährdung des Operationsverlaufs zur Folge haben könnten. Mit dem System können auch Ausfälle durch defekte Instrumente über den Zeitverlauf erkannt werden. Und bei Weiterentwicklungen können frühzeitig Verbesserungen am Produkt eingebaut werden.

Das erfindungsgemässe Überwachungssystem kann zum Beispiel auch für die Überwachung von medizinischen Einweg-Verbrauchsartikeln eingesetzt werden. Sie ermöglicht auch die automatische Überwachung der Lebensdauer des Artikels.

## Patentansprüche

1. Verfahren zum Überwachen des Zustands von Instrumenten und Werkzeugen insbesondere für chirurgische Eingriffe und ähnliche Einsätze, **dadurch gekennzeichnet, dass**
der Zustand der Instrumente/Werkzeuge anhand von für ihre Einsatzfähigkeit massgeblichen Parametern erfasst wird und die von diesen erhaltenen Daten zeitgleich oder zeitversetzt anhand von instrumentenspezifischen Sollvorgaben ausgewertet werden.

2. Verfahren nach Anspruch 1, **dadurch gekennzeichnet, dass** die Auswertung der von den Instrumenten/Werkzeugen erhaltenen Daten ein- oder mehrmals während, vor und/oder nach dem Einsatz in einem mit Sensoren durch Datenübertragungsmittel verbundenen Empfängern durchgeführt wird.

3. Verfahren nach Anspruch 2, **dadurch gekennzeichnet, dass** die Auswertungsergebnisse durch ein Mittel, wie ein Bildschirmgerät oder dergleichen, visualisiert werden können.

4. Verfahren nach einem oder mehreren der Ansprüche 1 bis 3, **dadurch gekennzeichnet, dass**
die von den Instrumenten/Werkzeugen ermittelten Daten an diesem selber oder auf einer Datenbank des Empfängers vorzugsweise aggregiert ausgewertet werden.

5. Verfahren nach einem oder mehreren der Ansprüche 1 bis 4, **dadurch gekennzeichnet, dass**
die von den Instrumenten/Werkzeugen ermittelten Daten von einem Computer oder einer ähnlichen zentralen Datenverarbeitungseinrichtung aufgezeichnet, gespeichert und vorzugsweise algorithmisch ausgewertet werden, indem sie dort mit den jeweils eingegebenen Sollvorgaben verglichen werden und über ihren weiteren Einsatz befunden wird.

6. Verfahren nach einem oder mehreren der Ansprüche 1 bis 5, **dadurch gekennzeichnet, dass**
der Computer zusätzlich zu den von den Instrumenten/Werkzeugen erhaltenen Daten weitere einsatzspezifische Daten wie Einsatzort, Einsatztermin und/oder Einsatzdauer des Eingriffes berücksichtigt.

7. Verfahren nach einem oder mehreren der Ansprüche 1 bis 6, **dadurch gekennzeichnet, dass**
der Computer eine Überlastung der Instrumente/Werkzeuge durch Aufzeichnen der auf ihn während des Einsatzes wirkenden Spannungen und Kräfte registriert.

8. Verfahren nach einem oder mehreren der Ansprüche 1 bis 7, **dadurch gekennzeichnet, dass**
der Computer eine Beschädigung oder das Ende der Lebensdauer der Instrumente/Werkzeuge registriert und dies durch ein optisches und/oder akustisches Signal angezeigt wird.

9. Verfahren nach einem oder mehreren der Ansprüche 1 bis 8, **dadurch gekennzeichnet, dass**
der Computer mittels eines Beschleunigungsmessers oder dergleichen das Herunterfallen der Instrumente/Werkzeuge aus der Hand des Operateurs oder zu Boden registriert.

10. Verfahren nach einem oder mehreren der Ansprüche 1 bis 9, **dadurch gekennzeichnet, dass**
der Computer einen Sterilisations- oder Reinigungsvorgang durch Temperatur-, Feuchte-, Impuls-, Dehnungs-, Überspannungs-, Überstrom- und EO-Konzentrationsmessung registriert.

11. Instrument/Werkzeug zum Ausführen des Überwachungsverfahrens nach einem oder mehreren der Ansprüche 1 bis 10, **dadurch gekennzeichnet, dass**
mindestens ein miniaturisierter Sensor (13, 14) oder ähnliches eingebaut ist, der vorzugsweise kontinuierlich jeweils ein oder mehrere der zu berücksichtigenden Parameter erfasst.

12. Instrument/Werkzeug nach Anspruch 11, **dadurch gekennzeichnet, dass**
der jeweilige Sensor (13, 14) die Daten auf den Empfänger mittels einer Funkverbindung, wie WLAN, Bluetooth, NFC oder ähnlichen Übertragungsmitteln übertragt.

13. Instrument/Werkzeug nach Anspruch 11 oder 12, **dadurch gekennzeichnet, dass**
der mindestens eine Sensor (13) in einer Einkapselung (16) des Instruments/Werkzeugs eingebaut ist.

14. Instrument/Werkzeug nach Anspruch 11 oder 12, **dadurch gekennzeichnet, dass**
der mindestens eine Sensor (14) unter einer Abdeckung (17) des Instruments/Werkzeugs eingebaut ist.
